# EUROPÄISCHE PATENTSCHRIFT

(11) **EP 3 218 069 B1**
(45) Veröffentlichungstag und Bekanntmachung des Hinweises auf die Patenterteilung: **29.01.2025**
(21) Anmeldenummer: 15798353.7
(22) Anmeldetag: 10.11.2015
(51) Int. Cl.: A61K 33/14, A61Q 19/00, A61K 9/00, A61K 45/06, A61K 31/194, A61K 33/04, A61K 33/20, A61K 33/40, A61P 17/02, A61P 31/02, A61P 29/00

(54) **KOSMETISCHES UND PHARMAZEUTISCHES MITTEL ZUR BEHANDLUNG VON WUNDEN UND/ODER BLUTUNGEN DER HAUT, DER SCHLEIMHÄUTE ODER DES ZAHNFLEISCHES, INSBESONDERE Z.B. VON HAUTRISSEN (RHAGADEN) ODER PARODONTITIS**
COSMETIC AND PHARMACEUTICAL AGENTS FOR TREATING WOUNDS AND/OR HEMORRHAGING OF THE SKIN, MUCOUS MEMBRANES OR THE GUM, IN PARTICULAR FOR EXAMPLE, SUPERFICIAL CRACKS (RHAGADEN) OR PERODONTITIS
AGENT COSMÉTIQUE ET PHARMACEUTIQUE POUR TRAITER DES PLAIES ET/OU DES HÉMORRAGIES CUTANÉES, MUQUEUSES OU GINGIVALES, NOTAMMENT P. EX. DES FISSURES DE LA PEAU (RHAGADES) OU UNE PARODONTITE

(30) Priorität: 11.11.2014 CH 17502014
(43) Veröffentlichungstag der Anmeldung: 20.09.2017
(73) Patentinhaber: Bogaert, Jean-Pierre, Monaco (MC)
(72) Erfinder: Bogaert, Jean-Pierre, Monaco (MC)
(74) Vertreter: Swisspat Riederer Hasler Patentanwälte AG
(86) Internationale Anmeldenummer: PCT/EP2015/076260
(87) Internationale Veröffentlichungsnummer: WO 2016/075169

(56) Entgegenhaltungen:
- EP-A1- 2 777 708
- EP-A1- 2 777 708
- EP-A1- 2 777 708
- WO-A1-2008/112940
- WO-A1-99/34773
- WO-A1-99/34773
- WO-A1-99/34773
- WO-A2-2004/000372
- WO-A2-2004/000372
- WO-A2-2004/000372
- US-A1- 2011 229 583
- US-A1- 2011 229 583
- US-A1- 2011 229 583
- US-B1- 6 280 775
- US-B1- 6 280 775
- US-B1- 6 280 775
- DUTT DR.POONAM ET AL: "Chlorhexidine - An antiseptic in periodontics", IOSR JOURNAL OF DENTAL AND MEDICAL SCIENCES, vol. 13, no. 9, 1 September 2014 (2014-09-01), pages 85 - 88, XP055894974, ISSN: 2279-0861, Retrieved from the Internet <URL:https://www.iosrjournals.org/iosr-jdms/papers/Vol13-issue9/Version-6/Q013968588.pdf> DOI: 10.9790/0853-13968588
- COSOLA SAVERIO ET AL: "The effectiveness of the information-motivation model and domestic brushing with a hypochlorite-based formula on peri-implant mucositis: A randomized clinical study", CLINICAL AND EXPERIMENTAL DENTAL RESEARCH, 22 October 2021 (2021-10-22), XP055894834, ISSN: 2057-4347, Retrieved from the Internet <URL:https://onlinelibrary.wiley.com/doi/full-xml/10.1002/cre2.487> DOI: 10.1002/cre2.487

## Beschreibung

### TECHNISCHES GEBIET DER ERFINDUNG

Die Erfindung umfasst ein Mittel zur Behandlung von Parodontitis.

### HINTERGRUND DER ERFINDUNG

Die Wundheilung kann in vielen Fällen heutzutage noch nicht optimal therapeutisch unterstützt und gefördert werden. Die Wissenschaft ist nach wie vor bemüht Mittel und Wege zu finden, Komplikation oder Infektion vorzubeugen, Heilungsverzögerungen zu verhindern und das kosmetische Resultat so optimal wie möglich zu gestalten. Noch immer gibt es Patienten die an chronischen oder nicht heilenden Wunden leiden.

Die Offenlegungsschrift EP 2 777 708 A1 stellt ein Mittel zur Behandlung von Aphthen bereit, welches eine fliessfähige, pastöse Haftzusammensetzung, welche geeignet ist, nach Applikation auf der Mundschleimhaut zu haften, und eine pharmazeutische Wirkzusammensetzung mit einer organischen oder anorganischen Chlorverbindung und wenigstens einem Oxidationsmittel enthält.

Die Patentschrift US 6,280,775 B1 offenbart eine Mundspülung zur Behandlung und Reduzierung von Zahnkrankheiten. Diese Mundspülung basiert auf einer Mischung einer Lösung eines wasserlöslichen Metallchlorits und einer Lösung beinhaltend Natriumpersulfat und Wasserstoffperoxid. Die daraus entstehende Zusammensetzung enthält Chlordioxid.

Die Offenlegungsschrift WO 99/34773 A1 beschreibt ein antimikrobielles Pflegemittel auf Basis einer Peroxycarbonsäure. Das Mittel wird insbesondere bereitgestellt in Form von Shampoos, Walzen Stifte, Gele, Spülungen und Mundpflegezusammensetzungen, wie Mundspülungen, Zahnpasten, Zahnpulver, Mundsprays, Kaugummis, Lutschtabletten, Sachets und Bleich Kits usw. für die Behandlung topisch behandelbarer mikrobieller Infektionen, insbesondere von Erkrankungen der Mundhöhle, wie Plaque, Gingivitis, Parodontitis und Mundgeruch, oder auch anaerobe Infektionen der Haut.

Die Offenlegungsschrift WO 2004/000372 A2 beschreibt eine Zusammensetzung zur Desinfektion von Zahnprothesen, Zahnbürsten, Rasiergeräten und dergleichen enthaltend eine salzförmige Chlorid- oder Bromidverbindung, mindestens ein Oxidationsmittel mit einem Oxidationspotential, welches in Lösung höher als das Oxidationspotential von Cl¹⁻/Cl⁰ resp. Br¹⁻/Br⁰ ist, und im Überschuss mindestens eine Säure zur Erzeugung eines pH-Wertes < 6 bei vollständig aufgelöster Zusammensetzung.

Die Offenlegungsschrift WO 00/19981 A1 offenbart eine antimikrobielle pharmazeutischen Zubereitungen (z.B. Lösungen, Gels, Salben, Cremes, Präparate mit verzögerter Freisetzung, etc.), welche Chlorit (beispielsweise ein Metallsalz eines Chlorits) in Kombination mit einer Peroxidverbindung (z.B. Wasserstoffperoxid) enthalten, für die Verwendung zur Desinfektion von Gegenständen oder Oberflächen (z.B. Kontaktlinsen, Abdeckungen, etc.), Antisepsis der Haut oder anderen Körperteilen, Verhinderung der Narbenbildung und/oder Behandlung und Prophylaxe von Erkrankungen der Haut oder Schleimhaut (z.B. Wunden, Verbrennungen, Infektionen, Erfrierungen, Ulzeration, Psoriasis, Akne oder andere Narben bildende Läsionen). Die Offenlegungsschrift US 2004/037891 A1 zeigt ebenfalls eine antimikrobielle Zubereitungen beinhaltend Chlorit in Kombination mit einer Peroxidverbindung. Wobei diese Zusammensetzung jedoch auch während der Lagerung bei Raumtemperatur stabil bleibt und kein Chlordioxid erzeugt.

Im Artikel "Chlorhexidine - An antiseptic in periodontics" von Dutt et al. (IOSR Journal of Dental and Medical Sciences, Bd. 13, Nr. 9, 1. September 2014, Seiten 85-88) wird festgestellt, dass Chlorhexidin das bislang wirksamste chemische Mittel gegen Plaque ist. Insbesondere wird die Verwendung von Chlorhexidin als Ergänzung zu mechanischen Therapien zur Eindämmung der Plaquebildung empfohlen.

### AUFGABE

Es ist eine Aufgabe der vorliegenden Erfindung, ein Mittel zur Schmerzlinderung und Wundheilungsförderung bereitzustellen. Insbesondere soll ein Mittel bereitgestellt werden, das einfach, vorzugsweise vom Patienten selbst, angewendet werden kann. Insbesondere soll ein Mittel gefunden werden, das bei topischer Anwendung zur Schmerzlinderung und Wundheilung beiträgt.

Weiter ist es eine Aufgabe der vorliegenden Erfindung, eine Mittel (Stoffgemisch) und eine Darreichungsform bereitzustellen, welche eine einfache, lokalisierte und nachhaltige Behandlung ermöglicht.

Zum Beispiel ist es Aufgabe ein Mittel zur Verwendung bei spröder Haut oder Hautrissen, z.B. an Fusssohle, Ferse, Zehe oder zwischen Zehen, an Hand, Finger oder zwischen Fingern, an Lippe, Mundwinkel, Nasenwinkel, in Gelenksbeugen oder an der Perianalregion, bereitzustellen. Es ist insbesondere Ziel ein Mittel bereitzustellen, mit welchem es gelingt, aufgesprungene Haut bzw. Hautrisse, insbesondere auch Einrisse an verhornten Hautstellen, zu mildern und zu heilen.

Es ist weitere eine Aufgabe der vorliegenden Erfindung, ein Mittel zur Verwendung in Mund- oder Rachenraum, insbesondere bei Zahnfleischproblemen oder Parodontitis, bereitzustellen.

Es ist eine weitere Aufgabe der vorliegenden Erfindung, eine Mittel zur topischen Anwendung bei Verletzungen der Schleimhaut in Mund- und Rachenraum, insbesondere des Zahnfleisches bzw. der Gingiva, bereitzustellen.

### ZUSAMMENFASSUNG

Die Erfindung bezieht sich auf ein Mittel zur Verwendung bei der Behandlung von Parodontitis, beinhaltend wenigstens eine salzförmigen Chloridverbindung ausgewählt aus der Gruppe der Alkali- oder Erdalkalimetallsalze und wenigstens ein Oxidationsmittel ausgewählt aus der Gruppe der Persulfatverbindungen.

Weiterbildungen und/oder vorteilhafte Ausführungsvarianten der Erfindung sind Gegenstand der abhängigen Patentansprüche. Die im Folgenden beschriebenen Verwendungen des erfindungsgemäßen Mittels, die nicht die Behandlung von Parodontitis betreffen, sind nicht Teil der Erfindung.

Das erfindungsgemässe Mittel eignet sich weiter insbesondere zur Verwendung
- bei der Behandlung von Rissen in der Haut, wie z.B. Rhagaden, insbesondere an Füssen und Händen, insbesondere an Fusssohle, Fusssohlenrand, Ferse, Zehen, Handfläche und/oder Finger,
- bei der Behandlung von schlecht heilenden Wunden, dazu gehören z.B. Wunden von Diabetikern, insbesondere Wunden an den Gliedern, z.B. an Beinen, Armen, Füssen, Händen, Zehen und Finger,
- bei der Behandlung von infizierten Wunden, insbesondere von bakteriell infizierten Wunden oder von mit Pilzen infizierten Wunden,
- zum Desinfizieren von Wunden,
- bei der Behandlung von blutenden Wunden, insbesondere z.B. bei Zahnfleischblutungen und/ oder
- bei der Behandlung von therapieresistenten (d.h. nicht heilenden) offenen Wunden an Haut oder Schleimhaut,
wobei diese Verwendungen für sich genommen nicht Teil der beanspruchten Erfindung sind.

Es lassen sich damit auch schwer heilenden, chronische oder nicht heilende Wunden, d.h. Wunden welche seit mehr als zwei Tagen, einer Woche, vier Wochen oder länger nicht heilen bzw. keine Verbesserung aufweisen, behandeln. Die Wundheilung wird allgemein beschleunigt. Das erfindungsgemässe Mittel eignet sich somit zur Wundheilungsbeschleunigung.

Im Weiteren wirkt das erfindungsgemässe Mittel als Entzündungshemmer oder Desinfektionsmittel, insbesondere bei Anwendung auf offenen Hautstellen oder auf Zahnfleisch. Zahnfleischbluten kann reduziert oder verhindert werden, insbesondere nach einer zahnärztlichen Behandlung und/oder bei Parodontitis. Das erfindungsgemässe Mittel eignet sich besonders zur Behandlung von Parodontitis, insbesondere bei der marginalen Parodontitis oder als Zusatzbehandlung (nach Zahnärztlichem Eingriff) bei der apikalen Parodontitis.

Das erfindungsgemässe Mittel wirkt weiter schmerzlindernd oder schmerzstillend bei den hierin genannten Haut- und Schleimhautdefekten bzw. Haut- und Schleimhautwunden, insbesondere nach einer zahnärztlichen Behandlung, wie z.B. einer Zahnreinigungsbehandlung, oder bei Hautrissen, insbesondere Rhagaden, und kann somit zusätzlich zur Schmerzlinderung eingesetzt werden.

Das erfindungsgemässe Mittel eignet sich insbesondere zur Verwendung bei der Behandlung von Parodontitis in Kombination mit einer Zahnreinigungsbehandlung, insbesondere einer Scaling-and-Root-Planing-(SRP)-Behandlung. Zweckmässigerweise wird das erfindungsgemässe Mittel im Anschluss an eine Zahnreinigungsbehandlung, insbesondere im Anschluss an eine Scaling-and-Root-Planing-(SRP)-Behandlung angewendet. Bevorzugt wird das erfindungsgemässe Mittel wenigstens gleichentags im Anschluss an eine Zahnreinigungsbehandlung oder Scaling-and-Root-Planing-(SRP)-Behandlung angewendet. Das erfindungsgemässe Mittel wird zweckmässigerweise auf das Zahnfleisch, insbesondere auf den Zahnfleischrand, aufgetragen, vorzugsweise um während 1 bis 3 Stunden einzuwirken. In den Tagen nach der Zahnreinigungsbehandlung oder Scaling-and-Root-Planing-(SRP)-Behandlung (vorzugsweise während mindestens drei, weiter bevorzugt mindestens 5 konsekutiven Tagen) kann zusätzlich die regelmässige Zahnpflege (z.B. wenigstens einmal pro Tag) mit einer wässrigen Lösung des erfindungsgemässen Mittels vorgenommen werden.

Aufgesprungene Haut bzw. Hautrisse, insbesondere spaltförmiger Einriss in der Haut, sind oft die Folge einer Überdehnung bei herabgesetzter Elastizität der Haut, entstanden z. B. bei Austrocknung, Kälte (klimatischen Einflüsse) oder Krankheiten (z.B. Hautkrankheiten) an Lippen, Mundwinkel, Nasenflügelansatz, Fußsohlen (insbesondere an Ferse oder an oder zwischen Zehen), Händen und/oder Fingern, Gelenksbeugen oder an der Perianalregion. Neben Umwelteinflüssen und Krankheiten können Hautrisse auch aufgrund andere Ursachen entstehen, z.B. aufgrund einer bakteriellen Infektion einer Hautverletzung. Eine Einreissung (d.h. ein Riss) in der Haut, insbesondere ein spaltförmiger Einriss in der Haut infolge Überdehnung bei herabgesetzter Elastizität, wird medizinisch als Rhagade und umgangssprachlich auch als Schrunde bezeichnet.

Einreissungen, d.h. Rhagaden, können auch entstehen. Die Einrisse sehen unschön aus, können schmerzhaft sein, können gegebenenfalls hygienisch problematisch sein und sind potentielle Eingangspforten für Krankheitserreger. Verzögert sich die Heilung können derartige Einrisse einschränkend wirken. Massnahmen zur Förderung der Abheilung sind daher wünschenswert.

Im Weiteren hat sich herausgestellt, dass das erfindungsgemässe Mittel auch bei andern Arten von Wunden zu einer Verbesserung und schlussendlich zur Heilung führt. Bei schlecht heilenden Wunden (z.B. aufgrund von Durchblutungsstörungen, Einnahme bestimmte Medikamente, Wundinfektionen, oder anderen Gründen) können sich ungünstig auf den Heilungsverlauf auswirken) ist das erfindungsgemässe Mittel vorteilhaft einsetzbar. Insbesondere sind Wunden von Diabetikern mit dem erfindungsgemässen Mittel erfolgreich behandelbar.

Weiter hat sich gezeigt, dass im Besonderen Zahnfleischverletzungen und Zahnfleischentzündungen rasch gelindert werden können.

Es hat sich herausgestellt, dass sich das erfindungsgemässe Mittel durch topisches Auftragen erfolgreich anwenden lässt.

Die wenigstens eine salzförmige Chloridverbindung und das wenigstens eine Oxidationsmittel definieren eine Wirkzusammensetzung, welche gegebenenfalls weitere Stoffe enthalten kann. Wird die Wirkzusammensetzung mit einer Haftungszusammensetzung, welche insbesondere als Paste, Creme, Salbe oder Gel vorliegt, vermengt ist die Behandlung besonders nachhaltig und intensiv aufgrund der besonderen Hafteigenschaften des Mittels. Ein (weiteres) Eindringen von Keimen aus der Umgebung wird verhindert.

Das erfindungsgemässe Mittel zeichnet sich weiter vorteilhafterweise dadurch aus, dass
- die Wirkzusammensetzung eine Carbonsäure, vorzugsweise eine Mono- oder Dicarbonsäure wie Zitronensäure, enthält,
- die Säure in einer solchen Menge in der Wirkzusammensetzung enthalten ist, dass die in einer bestimmten Menge einer wässerigen Lösung aufgelöste Wirkzusammensetzung - einen pH-Wert < 6, vorzugsweise < 5, und ganz besonders bevorzugt < 4.5 erzeugt,
- das Oxidationsmittel ausgewählt ist aus der Gruppe der Persulfatverbindungen, insbesondere dass als Oxidationsmittel eine Hydrogenperoxosulfatverbindung enthalten ist,
- die Chlorverbindung in Form eines Alkali- oder Erdalkalimetallsalzes vorliegt,
- die Chlorverbindung Chloramin B (N-Chlorbenzolsulfonamido-Natrium), Chloramin T (p-Toluolsulfonchloramin-Natrium), Dichloramin T (p-Toluolsulfondichloramid), Halazon (p-Dichlorsulfamylbenzoesäure), Dichlorcyanursäure, Trichlorcyanursäure, TCM (Trichlormelamin), 1,3-Dichlor-5,5-dimethylhydantoin, Dichlorglycoluril, Succinchlorimid oder Chloroazodin (N,N'-dichlorodiazo-dicarbonamidin) oder eine Mischung dieser Stoffe enthält (nicht entsprechend der Erfindung),
- die Wirkzusammensetzung wenigstens Kaliumhydrogenmonopersulfat (KHSOs) als Oxidationsmittel und, eine salzförmige Chloridverbindung, vorzugsweise aus der Gruppe der Alkalisalze, z.B. Kochsalz (NaCl), enthält,
- das Oxidationspotential des Oxidationsmittels in wässeriger Lösung mindestens im beanspruchten pH-Bereich höher als das Oxidationspotential von Cl¹⁻/Cl⁰ ist,
- die Wirkzusammensetzung Stoffe zur Lösungsbeschleunigung (Brausesalze), beispielsweise eine Carbonat oder Bicarbonat enthaltende Verbindung wie z.B. Natriumcarbonat oder Natriumbicarbonat, und eine mindestens stöchiometrische Menge Säure enthält,
- das Mittel ein Bindemittel, und optional Aroma-, Farb- und Hilfsstoffe, wie z.B. Stoffe zur Wasserenthärtung, Füllstoffe und dergleichen, enthält,
- die Wirkzusammensetzung mit der Haftungszusammensetzung vermengt oder vermischt ist, wobei die Haftungszusammensetzung geeignet ist, auf der Haut (d.h. äussere topische Anwendung) bzw. der Schleimhaut zu haften,
- die Haftungszusammensetzung
   - wenigstens Paraffine, Fette und/oder Öle
   - wenigstens ein wasserlösliches Polymer, vorzugsweise ausgewählt aus der Gruppe der Cellulosederivate und Polysaccharide, und
   - wenigstens ein Alkylvinylether/Maleinsäureanhydrid-Copolymer enthält,
- die Haftungszusammensetzung wenigstens einen Alkohol enthält,
- der wenigstens eine Alkohol bei Raumtemperatur und/oder Körpertemperatur als Flüssigkeit vorliegt, wobei Ethanol bevorzugt ist,
- der wenigstens eine Alkohol in einer Menge von bis zu 10 Gew.-% bezogen auf die Gesamtmenge der Haftungszusammensetzung zugegeben ist,
- das wenigstens eine wasserlösliche Polymer wenigstens ein Polysaccharid, insbesondere das Polysaccharid Xanthan beinhaltet,
- das wenigstens eine Polysaccharid in einer Menge von bis zu 20 Gew.-% bezogen auf die Gesamtmenge der Haftungszusammensetzung zugegeben ist,
- keine Fluoridsalze, vorzugsweise keine Fluoridverbindungen, enthalten sind,
- das Mittel in Form einer Creme, einer Paste oder eines Gels vorliegt, welche insbesondere geeignet ist, nach Applikation auf der Haut (d.h. bei äusserer topischer Anwendung), der Schleimhaut und/oder dem Zahnfleisch zu haften,
- zur Behandlung eine Schicht von mindestens 0.2 mm, bevorzugt mindestens 0.3 mm, weiter bevorzugt mindestens 0.5 mm, weiter bevorzugt mindestens 0.7 mm, weiter bevorzugt mindestens 1.0 mm, aufgetragen wird,
- das Mittel nach dem Auftragen auf eine zu behandelnde Stelle mit Wasser glattgestrichen wird,
- die Wirkzusammensetzung in Form eines pulverförmigen Feststoffgemisches vorliegt und als Pulver zur Anwendung bereitgestellt wird, und/ oder
- die Wirkzusammensetzung in Form eines pulverförmigen Feststoffgemisches der Haftungszusammensetzung beigemischt ist und als Creme, Paste oder Gel zur Anwendung bereitgestellt wird.

Im weiteren ist ein Mittel, insbesondere ein kosmetisches und/ oder pharmazeutisches Mittel, offenbart beinhaltend wenigstens eine salzförmige Chloridverbindung ausgewählt aus der Gruppe der Alkali- oder Erdalkalimetallsalze und wenigstens ein Oxidationsmittel ausgewählt aus der Gruppe der Persulfatverbindungen, dadurch gekennzeichnet, dass es in Form einer Creme, bzw. einer Salbe, einer Paste oder eines Gels vorliegt, welche geeignet ist, nach Applikation auf der Haut (d.h. bei äusserer topischer Anwendung), der Schleimhaut oder dem Zahnfleisch zu haften.

Ebenfalls offenbart ist eine Haftungszusammensetzung, welche sich besonders als Träger für ein Mittel mit einer Wirkzusammensetzung wie es hierin beschrieben ist eignet. Die Haftungszusammensetzung beinhaltet wenigstens ein Paraffin, Fett und/oder Öl, wenigstens ein wasserlösliches Polymer, vorzugsweise ausgewählt aus der Gruppe der Cellulosederivate und Polysaccharide, wenigstens ein Alkylvinylether/Maleinsäureanhydrid-Copolymer, und vorzugsweise wenigstens einen Alkohol.

Die Haftungszusammensetzung zeichnet sich weiter vorteilhafterweise dadurch aus, dass
- der Alkohol bei Raumtemperatur und/oder Körpertemperatur als Flüssigkeit vorliegt, wobei Ethanol besonders bevorzugt ist,
- der wenigstens eine Alkohol in einer Menge von bis zu 10 Gew.-% bezogen auf die Gesamtmenge der Haftungszusammensetzung zugegeben ist,
- das wenigstens eine wasserlösliche Polymer das Polysaccharid Xanthan beinhaltet,
- das wenigstens eine Polysaccharid in einer Menge von bis zu 20 Gew.-% bezogen auf die Gesamtmenge der Haftungszusammensetzung zugegeben ist.

Weiter offenbart ist ein Kit, insbesondere ein Kit zur Anwendung nach einer Zahnreinigungsbehandlung, beinhaltend zumindest eine erste Packungen einer ersten Dosierung des erfindungsgemässen Mittels und zumindest eine zweite Packung einer zweiten Dosierung des erfindungsgemässen Mittels. Vorzugsweise beinhaltet die zumindest erste Packung eine Kombination der Wirkzusammensetzung mit der Haftzusammensetzung. Die Wirkzusammensetzung oder vorzugsweise die Kombination der Wirkzusammensetzung mit der Haftzusammensetzung der zumindest ersten Packung wird zweckmässigerweise in Form einer Paste, einer Creme, einer Salbe oder eines Gels bereitgestellt. Diese erste Packung wird insbesondere direkt nach einer Zahnreinigungsbehandlung angewandt. Aufgrund der Haftzusammensetzung wird nach Auftragung des erfindungsgemässen Mittels eine zeitlich ausgedehnte und somit intensive Einwirkung, z.B. über 1-3 Stunden, erreicht.

Die zumindest eine zweite Packung ist vorzugsweise frei von der genannte Haftzusammensetzung oder beinhaltet eine geringere Menge der genannten Haftzusammensetzung als die ersten Packung. Die Wirkzusammensetzung der zumindest zweiten Packung wird zweckmässigerweise in Form eines Pulvers, einer Tablette, insbesondere in Form eines wasserlöslichen Pulvers bzw. einer wasserlöslichen Tablette, einer Paste, insbesondere einer Zahnpaste, oder einer Lösung, insbesondere einer wässrigen Lösung, bereitgestellt. Die genannte Lösung kann relativ stark konzentriert sein und ist gegebenenfalls in Wasser weiter verdünnbar. Die zumindest eine zweite Packung ist mehrfach, bevorzugt zumindest dreimal, weiter bevorzugt zumindest fünfmal, im Kit enthalten. Je eine der zweiten Packungen kann an je einem der der Behandlung nachfolgenden Tage angewendet werden - zum Beispiel einmal pro Tag indem die jeweilige Dosierung, z.B. per Zahnbürste, aufgetragen wird.

Zusätzliche Merkmale, Vorteile und Ziele der vorliegenden Erfindung ergeben sich aus der nachfolgenden Beschreibung. Die im Folgenden angeführten vorteilhaften Ausführungsvarianten führen allein oder in Kombination miteinander zu weiteren Verbesserungen des erfindungsgemässen Mittels.

### BESCHREIBUNG

Die Wirkzusammensetzung zeichnet sich dadurch aus, dass sie wenigstens eine salzförmige Chloridverbindung, welche ausgewählt ist aus der Gruppe der Alkali- oder Erdalkalimetallsalze, und wenigstens ein Oxidationsmittel, welches ausgewählt ist aus der Gruppe der Persulfatverbindungen, enthält. Diese Wirkzusammensetzung hat den Vorteil, dass nach der Applikation des Mittels auf einer verletzten und/oder entzündeten Stelle sich durch den Kontakt mit Feuchtigkeit (d.h. Wasser) Chloroxid oder Hypochlorit bildet, welches für die heilende Wirkung des Mittels verantwortlich zu sein scheint. Die Haftungszusammensetzung - insoweit vorhanden - bildet dabei eine Barriereschicht, in welcher gleichzeitig die pharmazeutisch aktiven Stoffe enthalten sind. Diese entfalten ihre Wirkung jedoch erst, wenn Feuchtigkeit zu diesen gelangt.

Im Rahmen der vorliegenden Beschreibung sind mit dem Begriff "Wirkzusammensetzung" jeweils Komponenten des erfindungsgemässen Mittels gemeint, welche alleine oder in Kombination kosmetische und pharmazeutische Wirksamkeit zeigen. Unter dem Begriff "Haftungszusammensetzung" sind hingegen Komponenten gemeint, welche alleine oder in Kombination als Träger (carrier) und Applikationsmedium für die Wirkzusammensetzung dienen können. Die Haftungszusammensetzung hat aber auch den Zweck, eine Barriereschicht über der zu behandelnden Stelle (z.B. Einreissung bzw. der Verletzung und/oder gegebenenfalls Entzündung) zu bilden und die Wirkstoffe erst allmählich freizusetzen. Eine lokalisierte Auftragung und Haftung des erfindungsgemässen Mittels aufgrund der Haftungszusammensetzung ermöglicht dabei einen lokalen und somit zielgenauen Einsatz der Wirkstoffe.

Die Haftungszusammensetzung hat zweckmässigerweise eine Haftfestigkeit von mehr als 5 N [Newton], weiter bevorzugt von mehr als 7 N (insbesondere bei Messung nach ISO Norm 10873:2010, insbesondere bei einem 1-Minuten-Test und/oder einem 10-Minuten-Test bei 25°C).

Die Haftungszusammensetzung hat zweckmässigerweise eine Viskosität im Bereich von 20 bis 50 mPa s [Millipascal-Sekunde], weiter bevorzugt von 25 bis 45 mPa s (insbesondere Messung mit dem Brookfield-Viskosimeter, zweckmässigerweise bei 100 RPM mit Spindel (Zylinder) Nr. 4 und bei 25°C, insbesondere einer Mischung von 5 g Propylenglycol und 1 g der erfindungsgemässen Paste).

Die Haftungszusammensetzung hat zweckmässigerweise einen Konsistenzwert im Bereich von 10 bis 50 mm [Millimeter], weiter bevorzugt von 20 bis 40 mm (insbesondere bei Messung nach BE EN 30193-1:1994, insbesondere bei einem Gewicht 1 kg bei 25°C).

Das Chloratom kann in der Ausgangsverbindung entweder in Form eines Alkali- oder Erdalkalimetallsalzes (das ist eine in wässeriger Lösung Chloridionen freisetzende Verbindung) vorliegen. Auch kann eine Mischung der vorgenannten chlorhaltigen Stoffe eingesetzt sein.

Das erfindungsgemässe Mittel zeichnet sich vorzugsweises dadurch aus, dass keine Fluoridsalze, vorzugsweise insgesamt keine Fluoridverbindungen, enthalten sind.

Es ist ein Oxidationsmittel eingesetzt, welches in wässeriger Lösung von der Chlorverbindung Chlor oder Chloroxid abzuspalten vermag. Geeignete Oxidationsmittel sind beispielsweise Persulfatverbindungen, insbesondere Hydrogenperoxosulfat-verbindungen. In Verbindung mit wenigstens einem geeigneten Oxidationsmittel kann durch Reaktion mit gelösten Chloridionen (Cl-) Chlor (Cl₂) oder Dichloroxid (Cl₂O⁻) gebildet werden. Der Vorteil einer derartigen Wirkzusammensetzung ist, dass das Chlor nach und nach freigesetzt wird. Vorteilhaft ist das Oxidationspotential des Oxidationsmittels in Lösung mindestens beim bevorzugten pH-Bereich < 6, resp. < 5 höher als das Oxidationspotential von Cl⁻/Cl^{o}, damit die erwünschte Reaktion eintreten kann.

Obwohl grundsätzlich unterschiedliche Oxidationsmittel eingesetzt werden können, ist in der Wirkzusammensetzung als Oxidationsmittel eine Persulfatverbindung, insbesondere eine Hydrogenperoxosulfatverbindung enthalten. Persulfatverbindungen umfassen Peroxomonosulfatverbindungen (d.h. Verbindungen beinhaltend das Anion [SO₅]²⁻) und Peroxodisulfatverbindungen (d.h. Verbindungen beinhaltend das Anion [S₂O₈]²⁻). Beispiele dieser Verbindungen sind Peroxomonosulfate wie Na₂SO₅ oder KHSO₅ und Peroxodisulfate wie Na₂S₂O₈. Besonders bevorzugt als Oxidationsmittel sind die Hydrogenperoxosulfat-verbindungen. Ein vorteilhaftes Mittel erhält man mit Kaliumhydrogenmonopersulfat (KHSOs) als Oxidationsmittel und einer salzförmigen Chloridverbindung, vorzugsweise aus der Gruppe der Alkalisalze, z.B. Kochsalz (NaCl). Dieses hat sich als besonders wirksam erwiesen.

Zweckmässigerweise enthält das erfindungsgemässe Mittel, insbesondere die Wirkzusammensetzung, eine Carbonsäure, vorzugsweise eine Mono- oder Dicarbonsäure wie Zitronensäure. Diese organischen Carbonsäuren sorgen für einen sauren pH-Wert, welcher die Wirksamkeit des Mittels noch verbessern kann.

Eine vorteilhafte Wirkzusammensetzung, welche kostengünstig herstellbar ist, enthält Kaliumhydrogenmonopersulfat (KHSOs), Kochsalz (NaCl) und eine Säure, vorzugsweise in Form einer Monocarbonsäure oder einer Dicarbonsäure wie Oxalsäure, Weinsäure, Bernsteinsäure oder Zitronensäure. Es ist vorzugsweise eine solche Menge Säure in der Wirkzusammensetzung enthalten, dass nach dem Auflösen der Wirkzusammensetzung im wässerigen Milieu (5 g Wirkzusammensetzung in 50 ml Wasser) ein pH-Wert < 5, vorzugsweise < 4.5 erreicht ist. Zitronensäure ist physiologisch völlig unbedenklich, wird in der Natur rasch abgebaut und daher bevorzugt in der Wirkzusammensetzung eingesetzt.

Die Wirkzusammensetzung kann zusätzlich keimtötende Verbindungen, wie z.B. Kaliummonopersulfat, Kalium-Carbonate, Natriumperoxycarbonat, Natriumbenzoat, Subtilisin, Kaliumbenzoat, Chlorhexidin, eine Kombination von Chlorhexidin und Thymol, Cetylpyridiniumchlorid, Halogen abspaltende Verbindungen wie PVP-Jod und Cyanursäurechlorid, und/ oder Formaldehyd abspaltende Verbindungen wie Paraformaldehyd und/oder Methylolverbindungen etc. enthalten. Vorteilhaft sind zudem schmerzstillende Wirkstoffe wie beispielsweise Lidocain, Polidocanol oder Benzydamin eingesetzt.

Zweckmässigerweise enthält die Wirkzusammensetzung ein Bindemittel, und optional Aroma-, Farb- und Hilfsstoffe wie Stoffe zur Wasserenthärtung, Füllstoffe und dergleichen. Als Bindemittel kann beispielsweise modifizierte Maisstärke, mikrokristalline Cellulose, Sorbitol, hydrierte Soyatriglyceride, Polyethylenglykol wie PEG 180, PEG 150, PEG 75, Polyvinylpyrrolidone, ein Copolymer von Ethylenoxid und Propylenoxid, Polyvinylpyrolidon oder ein Copolymer von Polyvinylpyrolidon Vinylacetat, etc. eingesetzt werden. Der gewichtsmässige Anteil des Bindemittels beträgt zweckmässigerweise maximal ungefähr 30 Gew.-% und liegt vorzugsweise zwischen 5 und 30 Gew.-%, weiter vorzugsweise zwischen 5 und 25 Gew.-% des Gesamtgewichts der Wirkzusammensetzung, Wird eine Haftungszusammensetzung in Verbindung mit der Wirkzusammensetzung verwendet kann auf ein weiteres Bindemittel, wie es oben beschreiben ist, gegebenenfalls verzichtet werden, da die hierin beschriebene Haftungszusammensetzung insgesamt die Wirkzusammensetzung aufnimmt und/oder mindestens eines ihrer Bestandteile die Bindefunktion für die Wirkzusammensetzung übernimmt.

Die Haftungszusammensetzung enthält vorzugsweise wenigstens ein wasserlösliches Polymer, vorzugsweise ausgewählt aus der Gruppe der Cellulosederivate, und wenigstens ein Alkylvinylether/Maleinsäureanhydrid-Copolymer, und optional raffinierte Paraffine (Vaselin), Fette und/oder Öle. Die Haftungszusammensetzung kann weiter optional zinkhaltige Substanzen insbesondere Zinkoxid, Siliziumdioxid und Polyethylenglykol enthalten. Mögliche derartige Zusammensetzungen und ähnliche Zusammensetzungen, welche als Haftungszusammensetzung eingesetzt werden können, sind in den folgenden Patenten und Patentanmeldungen erwähnt: US Patentanmeldung Nr. 13/574,230, US Patentanmeldung Nr. 13/574,224 , US 4,758,630 und US 5,561,177, EP Patente EP 2 525 768 und EP 2 525 769.

Vorteilhaft ist die Wirkzusammensetzung in Form eines pulverförmigen Feststoffgemisches der Haftungszusammensetzung beigemischt. Diese Form der Beimengung kann einen retardierenden Effekt auf die Wirkstoffentfaltung haben, indem z.B. erst durch den allmählichen Kontakt mit dem Speichel die Auflösung der pulverförmigen Wirkzusammensetzung erfolgt.

In der Wirkzusammensetzung kann optional ein Tensid enthalten sein. Grundsätzlich können unterschiedliche, dem Fachmann bekannte Tenside eingesetzt werden. Wenn Carbonsäuren eingesetzt sind, so sollen die Tenside bei einem pH-Wert kleiner 7 stabil sein. Unter "stabil" soll in diesem Zusammenhang verstanden werden, dass max. 10% des eingesetzten Tensids in Lösung bei Raumtemperatur innerhalb von 30 Minuten zerfallen. Vorzugsweise werden als Tenside Fettalkohol-Polyglykoläther, Alkylbenzolsulfonate, Alkylsulfonate eingesetzt. Besonders bewährt haben sich anionische Tenside, vorzugsweise aus der Gruppe der Alkylethersulfate, wie Fettalkoholethersulfat-Alkalisalze, z.B. Natrium-n-alkyl-C₁₂₋₁₄-diglykoläthersulfat oder Natrium-n-alkyl-C₁₂₋₁₄-digly-koläthersulfat. Weiter eignen sich beispielsweise Na-Laurylsulfat, Na-Laurylsulfoacetat, Trinatriumphosphat.

Eine beispielhafte Wirkzusammensetzung enthält folgende Komponenten:

| **Stoff** | **in Gew.-proz. (%)** | **bevorzugte Bereiche in Gew.-proz. (%)** |
|---|---|---|
| Zitronensäue | 30 | 15 bis 50 |
| Laurylsulfat (insb. NatriumLaurylsulfat) | 15 | 2 bis 30 |
| Natrium-bicarbonat und/oder Natriumcarbonat | 20 | 10 bis 40 |
| Natriumchlorid | 10 | 4 bis 20 |
| Kaliumhydrogen-Monopersulfat | 5 | 1 bis 10 |
| Bindemittel | 20 | 0 bis 30 |

Zur Herstellung des kosmetisch und/oder pharmazeutisch wirksamen Mittels (Stoffgemisches) wird die Wirkzusammensetzung zweckmässigerweise in einer Haftungszusammensetzung (d.h. einer Haftcreme oder einem Haftgel) dispergiert und fein verteilt, bis eine homogene Mischung erreicht ist. Dabei werden die Haftungszusammensetzung und die Wirkzusammensetzung in einem gewichtsmässigen Verhältnis zwischen 99 : 1 und 80 : 20, vorzugsweise zwischen 98 : 2 und 88 : 12 miteinander gemischt.

Die Haftungszusammensetzungen, welche als Trägermaterial für die Wirkzusammensetzung (i.e. für die kosmetisch und/oder pharmazeutisch wirksamen Komponenten bzw. Zusammensetzung) dient, nimmt die Wirkzusammensetzung bzw. deren Bestandteile auf, z.B. durch Mischen der Haftungszusammensetzungen mit der Wirkzusammensetzung zu einem Stoffgemisch, wodurch die Adhäsionseigenschaften der Haftungszusammensetzung mit der kosmetischen und pharmazeutischen Wirkung der Wirkzusammensetzung vereint wird.

Im Folgenden wird eine besonders bevorzugte Haftungszusammensetzung vorgestellt. Die besonders bevorzugte Haftungszusammensetzung basiert auf wenigstens einem Öl oder Fett oder einem Gemisch davon, vorzugsweise pflanzlicher Art, und wenigstens einem Alkylvinylether/Maleinsäureanhydrid-Copolymer. Vorteilhafterweise liegt das Alkylvinylether/Maleinsäureanhydrid-Copolymer zum Teil als Säure, Ester und/oder Salz vor. Üblicherweise sind die Kationen der Salze ausgewählt aus der Gruppe bestehend aus Calcium-, Kalium-, Natrium-, Magnesium-, Aluminium-, Zinksalzen und Gemischen davon, insbesondere aus der Gruppe bestehend aus Ca²⁺, K⁺, Na⁺, Mg²⁺, Al³⁺ und/oder Zn²⁺. Als das Alkylvinylether/Maleinsäureanhydrid-Copolymer wird insbesondere ein Methylvinylether/Maleinsäureanhydrid-Copolymer verwendet. Das Methylvinylether/Maleinsäureanhydrid-Copolymer liegt, zum Beispiel als Salz, Ester und/oder Säure, in einer Menge von 20-45 Gew.-% bezogen auf die Gesamtmenge der Haftungszusammensetzungen vor. Vorteilhafterweise liegt das Methylvinylether/Maleinsäureanhydrid-Copolymer, zum Beispiel als Salz und/ oder Säure, in einer Menge von 25-45 Gew.-% und bevorzugt 25-40 Gew.-% und weiter bevorzugt 28-40 Gew.-% bezogen auf die Gesamtmenge der Haftungszusammensetzungen vor.

Insbesondere beinhaltet die Haftungszusammensetzung weiter wenigstens ein wasserlösliches Polymer ausgewählt aus der Gruppe der Cellulosederivate. Die Cellulosederivate sind wasserlösliche Polymere vorzugsweise ausgewählt aus der Gruppe bestehend aus Methylcellulose, Carboxymethylcellulose, Sodiumcarboxymethylcellulose, Hydroxypropylmethylcellulose und Gemischen davon. Bevorzugt wird Carboxymethylcellulose, insbesondere Natriumcarboxymethylcellulose, verwendet. Das wasserlösliche Polymer ausgewählt aus der Gruppe der Cellulosederivate liegt z.B. in einer Menge von bis zu 45 Gew.-% bezogen auf die Gesamtmenge der Haftungszusammensetzungen vor. Vorzugsweise liegt das wasserlösliche Polymer ausgewählt aus der Gruppe der Cellulosederivate in einer Menge von 2 bis 40 Gew.-% bezogen auf die Gesamtmenge der Haftungszusammensetzungen vor.

Vorzugsweise beinhaltet die Haftungszusammensetzung weiter wenigstens ein wasserlösliches oder quellbares Polymer ausgewählt aus der Gruppe der Polysaccharide, insbesondere Xanthan. Dieses dient als Verdicker. Bevorzugt sind Polysaccharide, welche eine Strukturviskosität aufweisen, also scher verdünnend wirken. Insbesondere Xanthan quillt in wässriger Lösung, erhöht dadurch die Viskosität des Mediums und wirkt scherverdünnend. Polysaccharide, insbesondere Xanthan, liegt vorzugsweise in einer Menge von 0 bis 20 Gew.-%, weiter bevorzugt von 2 bis 20 Gew.-% bezogen auf die Gesamtmenge der Haftungszusammensetzungen vor.

Vorteilhafterweise ist die Summe der Menge an Cellulosederivaten und Polysacchariden im Bereich von 2 bis 40 Gew.-% bezogen auf die Gesamtmenge der Haftungszusammensetzungen. Als besonders vorteilhaft hat sich ein Gewichtsverhältnis von Polysacchariden, wie z.B. Xanthan, zu Cellulosederivaten von mindestens 1:1, weiter bevorzugt von mindestens 2:1, erwiesen. Insoweit im Rahmen dieser Anmeldung die Cellulosederivate den Polysacchariden gegenübergestellt werden, werden die Cellulosederivate nicht zur Gruppe der Polysaccharide gezählt.

Optional beinhaltet die Haftungszusammensetzung weiter einen flüssigen Alkohol, insbesondere Ethanol. Der Alkohol liegt vorzugsweise in einer Menge von 0 bis 10 Gew.-%, und weiter bevorzugt von 2 bis 8 Gew.-%, und weiter bevorzugt von 3 bis 7 Gew.-%, bezogen auf die Gesamtmenge der Haftungszusammensetzung vor. Durch Zugabe des genannten Alkohols wird eine verbesserte Bindung der Haftungszusammensetzung erzielt.

Die Haftungszusammensetzung kann zumindest eine weitere Substanz ausgewählt aus der Gruppe bestehend aus Trihydroxystearin, Siliziumdioxid und Phosphoglyceriden enthalten.

Die Haftungszusammensetzung beinhaltet vorzugsweise Trihydroxystearin in einer Menge von 0.001-5.0 Gew.-%, und weiter bevorzugt von 0.1-4.5 Gew.-%, und weiter bevorzugt von 0.5-4.0 Gew.-%, bezogen auf die Gesamtmenge der Haftungszusammensetzung. Durch Zugabe von Trihydroxystearin werden die Hafteigenschaften der Haftungszusammensetzung und somit des gesamten erfindungsgemässen Mittels (Stoffgemisches) verbessert.

Die Haftungszusammensetzung beinhaltet gegebenenfalls Siliziumdioxid in einer Menge von 0.001-5 Gew.-%, weiter bevorzugt von 0.1-4 Gew.-% , bezogen auf die Gesamtmenge der Haftungszusammensetzung. Die Fliessfähigkeit und die Konsistenz der Haftungszusammensetzung können durch Zugabe von Siliziumdioxid positiv beeinflusst werden. Durch Siliziumdioxid kann eine Verflüssigungsneigung wirksam verhindert werden.

Die Haftungszusammensetzung beinhaltet gegebenenfalls Phosphoglyceride bevorzugt in einer Menge von 0.001-4 Gew.-%, weiter bevorzugt von 0.01-3 Gew.-% und weiter bevorzugt von 0.1-2 Gew.-% bezogen auf die Gesamtmenge der Haftungszusammensetzung. Die Phosphoglyceride sind insbesondere aus der Gruppe der Lecithine ausgewählt, zweckmässigerweise z.B. Soyalecithin.

Eine bevorzugte Haftungszusammensetzung zeichnet sich dadurch aus, dass zumindest ein Lecithin und Trihydroxystearin in Kombination darin vorliegen, gegebenenfalls Lecithin, Trihydroxystearin und Siliziumdioxid in Kombination vorliegen. Dabei liegt vorteilhafterweise Lecithin in einer Menge von 0.001-4 Gew.-%, weiter bevorzugt von 0.001-3 Gew.-%, besonders bevorzugt von 0.01-2 Gew.-%, und Trihydroxystearin in einer Menge von 0.001-5 Gew.-%, weiter bevorzugt von 0.1-4.5 Gew.-%, besonders bevorzugt von 0. 5-4 Gew.-% bezogen auf die Gesamtmenge der Haftungszusammensetzung vor. Werden pflanzliche Öl bzw. Fett, insbesondere mit einen gewissen minimalen ungesättigten Fettsäureanteil, für die Haftungszusammensetzung verwendet, dann werden vorteilhafte Hafteigenschaften besonders dann erzielt, wenn die Haftungszusammensetzung Substanzen ausgewählt aus der Gruppe bestehend aus Phosphoglyceriden und Trihydroxystearin beinhaltet.

Alternativ liegt Siliziumdioxid oder optional in Kombination mit Trihydroxystearin und/oder einem Phosphoglycerid, insbesondere Lecithin, vor. Neben dem Siliziumdioxid führen auch Trihydroxystearin und Polyethylenglycol zu stabilen Haftungszusammensetzungen. Diese Substanzen liefern sowohl für Zusammensetzungen auf Basis pflanzlicher Öle bzw. Fette sowie auf Basis mineralischer Öle bzw. Fette gute Ergebnisse.

Es ist bevorzugt, dass die Gesamtmenge der enthaltenen Trihydroxystearin, Siliziumdioxid und Phosphoglyceride in Summe die Menge von maximal 15 Gew.-%, bevorzugt maximal 12 Gew.-%, weiter bevorzugt maximal 10 Gew.-% bezogen auf die Gesamtmenge der Haftungszusammensetzung nicht übersteigt.

Eine besonders bevorzugte Haftungszusammensetzung zeichnet sich dadurch aus, dass ein pflanzliche Öl bzw. Fett zu wenigstens 25 Gew.-%, bevorzugt zu wenigstens 30 Gew.-%, bezogen auf die Gesamtmenge der Haftungszusammensetzung enthalten ist, wobei der Fettsäuregehalt des pflanzlichen Öls bzw. Fetts bevorzugt zu wenigstens 20 Gew.-%, weiter bevorzugt zu wenigstens 30 Gew.-%, weiter bevorzugt mehrheitlich, weiter bevorzugt zu wenigstens 65 Gew.-%, weiter bevorzugt zu wenigstens 80 Gew.-%, aus höheren Fettsäuren mit einer Kettenlänge von 16-18 C-Atomen besteht.

Eine besonders bevorzugte Haftungszusammensetzung zeichnet sich weiter dadurch aus, dass pflanzlichen Öls bzw. Fetts verwendet werden, deren Fettsäuregehalt insbesondere zumindest zu 20 Gew.-%, vorzugsweise 40 Gew.-%, weiter bevorzugt 50 Gew.-%, weiter bevorzugt 60 Gew.-%, weiter bevorzugt 70 Gew.-%, weiter bevorzugt 80 Gew.-% aus ungesättigte Fettsäuren besteht. Dies bringt den Vorteil mit sich, dass die Haftungszusammensetzung beim Verschlucken besonders verträglich ist, was insbesondere bei Anwendung an Kindern von Vorteil sein kann.

Die Menge an Pflanzenöl bzw. Fett beeinflusst die Konsistenz der Haftungszusammensetzungen. Wird zu wenig zugegeben, kann die Haftungszusammensetzung und somit das gesamte Stoffgemisch (beinhaltend Wirkzusammensetzung und Haftungszusammensetzung) eine körnig trockene Konsistenz annehmen. Die Zugabe von Siliziumdioxid, Trihydroxystearin und Phosphoglyceriden kann dem entgegenwirken.

Eine bevorzugte Haftungszusammensetzung beinhaltet beispielsweise bezogen auf die Gesamtmenge der Haftungszusammensetzung
a) 25-60 Gew.% wenigstens eines Öls und/oder Fetts, bevorzugt pflanzlicher Art,
b) 2-40 Gew.-% wenigstens eines wasserlöslichem Polymers ausgewählt aus der Gruppe der Cellulosederivate und Polysaccharide,
c) 25-45 Gew.-% des wenigstens einen Alkylvinylether/Maleinsäureanhydrid-Copolymers,
d) 0-10 Gew.-% oder gegebenenfalls bis zu 10 Gew.-% eines Alkohols,
e) 0-5 Gew.-% oder gegebenenfalls bis zu 5 Gew.-% Trihydroxystearin,
f) 0-4 Gew.-% oder gegebenenfalls bis zu 4 Gew.-% Phosphoglyceride,
g) 0-5 Gew.-% oder gegebenenfalls bis zu 5 Gew.-% Siliziumdioxid, und
h) 0-10 Gew.-% weitere Zusatzstoffe.

Zu den weiteren Zusatzstoffen zählen z.B. insbesondere Aromastoffe (wie z.B. Menthol, Citrus Limone, Pfefferminzaroma und Menthyl-Lactat), Lactose-Monohydrat, Zinkoxid (ZnO), Petrolatum, Paraffine und Vaseline. Weitere mögliche Zusatzstoffe sind z.B. Antioxidantien, Farbstoffe, Stabilisatoren, Verdicker, Emulgatoren, etc. Ein Stoff kann hierbei mehreren Wirkungsgruppen angehören, bzw. mehrere Wirkungen entfalten.

Zinkoxid kann die Haftkraft und die Haftungsdauer beeinflussen, insbesondere erhöhen. Bei Anwendungen in Mund- und Rachenraum sollte vorzugsweise auf Zinkoxid verzichtet werden. Es wird im Allgemeinen bevorzugt den Zinkgehalt auf eine Obergrenze von maximal 1 Gew.-% auf die Gesamtmenge der Haftungszusammensetzung zu beschränken bzw. bevorzugterweise keine Zinkzusätze zu verwenden.

Die Zugabe von Paraffinen und Vaseline ist optional. Bevorzugterweise wird der Gehalt an Paraffinen und Vaseline insgesamt auf maximal 3 Gew.-%, weiter bevorzugt auf maximal 1 Gew.-% auf die Gesamtmenge der Haftungszusammensetzung beschränkt. Vorteilhafterweise wird, insbesondere bei Anwendungen in Mund- und Rachenraum, kein Paraffin und Vaseline zugegeben.

Die Zugabe von Petrolatum ist optional. Bevorzugterweise wird der Gehalt an Petrolatum auf maximal 2 Gew.-%, weiter bevorzugt auf maximal 0.5 Gew.-% auf die Gesamtmenge der Haftungszusammensetzung beschränkt. Vorteilhafterweise wird, insbesondere bei Anwendungen in Mund- und Rachenraum, kein Petrolatum zugegeben.

Die Zugabe von Lactose-Monohydrat ist optional. Sie kann als Träger für Aroma und/ oder die Wirksubstanz dienen. Sie fördert die Konsistenz des Gemisches und führt insbesondere zu einer cremigeren Konsistenz. Bevorzugterweise wird der Gehalt an Lactose-Monohydrat insgesamt auf maximal 1 Gew.-%, weiter bevorzugt auf maximal 0,5 Gew.-% auf die Gesamtmenge der Haftungszusammensetzung beschränkt.

Zur Anwendung durch den Benutzer/Patienten wird ein Pfropfen des viskosen Mittels auf die zu behandelnde Stelle (insbesondere eine Wunde und gegebenenfalls Entzündung) aufgetragen und darüber verteilt, damit auch die Randzone der zu behandelnden Stelle gut abgedeckt ist. Das Mittel bildet auf der zu behandelnden Stelle eine Barriere- oder Schutzschicht. Gleichzeitig entfalten die in der Schutzschicht vorhandenen pharmazeutisch aktiven Ingredienzen ihre Wirkung. Durch die von Haut, Schleimhaut und/oder Wunde stammenden Feuchte und einer gegebenenfalls in der Umgebung vorhandenen Feuchte werden die im Mittel vorhandenen pulverförmigen Komponenten gelöst, sodass diese miteinander reagieren können. Weil diese Komponenten nur langsam in Lösung gehen, kann eine gute Langzeitwirkung erzielt werden.

Ein Mittel zur topischen Behandlung umfasst vorteilhafterweise eine fliessfähige, pastöse Haftungszusammensetzung, welche geeignet ist, nach Applikation auf der zu behandelnden Stelle zu haften, und eine pharmazeutische Wirkzusammensetzung mit einer salzförmigen Chloridverbindung ausgewählt aus der Gruppe der Alkali- oder Erdalkalimetallsalze und wenigstens einem Oxidationsmittel ausgewählt aus der Gruppe der Persulfatverbindungen. Wenn die organische oder anorganische Chlorverbindung und das Oxidationsmittel in Lösung gehen, bildet sich Chlor oder Chloroxid, welches sich überraschenderweise besonders wirksam bei der Behandlung von Wunden und gegebenenfalls Entzündungen verschiedenster Art herausgestellt hat. Vorteilhaft enthält die Wirkzusammensetzung eine organische Carbonsäure, um einen pH-Wert < 6 einzustellen. Nachfolgend wird die Erfindung anhand von Beispielen erläutert.

### KURZBESCHREIBUNG DER FIGUREN

- Fig. 1: Biofilm inhibierende und zerstörende Wirkung einer Salbe gemäß der Erfindung.
- Fig. 2: Mikrobielle Zahnfleischinfektion über die Behandlungsperiode.
- Fig. 3: Zusammenfassung von Plaque-Index-Progression über die Behandlungsperiode.
- Fig. 4: Zusammenfassung der Gingival-Index-Progression über die Behandlungsperiode.
- Fig. 5: Zusammenfassung der parodontalen Blutungsindexprogression über die Behandlungsperiode.
- Fig. 6: Halitosis-Gefühl nach 11 Tagen.

### ANWENDUNGSBEISPIELE

### Anwendungszusammensetzung A:

Die folgende Tabelle zeigt ein erfindungsgemässes Mittel in Form einer Paste, Salbe oder Creme zur topischen Anwendung auf spröden oder eingerissenen Hautstellen, insbesondere zur Anwendung auf Fusssohle, Ferse, Zehe, Hand, Handfläche, Finger, Mundwinkel, Nasenwinkel.

| **Inhaltsstoffe** | | **CAS Nr.** | **Gewichtsprozent (Gew.-%)** |
|---|---|---|---|
| | Ca/Na PVM/MA Co-polymer | 62386-95-2 | 37,978 |
| | Olea Europa (Olivenöl) | 8001-25-0 | 30,058 |
| | Xanthan | 11138-66-2 | 11,116 |
| | Alkohol | 64-17-5 | 5,187 |
| | Carboxymethylcellulose (CMC) | 9004-32-4 | 3,705 |
| | Trihydroxystearin | 139-44-6 | 3,578 |
| | Siliziumdioxid | 68611-44-9, 112945-52-5 | 3,110 |
| | Lezithin | 8002-43-5 | 1,017 |
| | Zinkoxid | 1314-13-2 | 0,926 |
| | Paraffinum Liquidum | 8042-47-5 | 0,642 |
| | Zitronensäure | 77-92-9 | 0,599 |
| | Natriumlaurylsulfat | 151-21-3 | 0,400 |
| | Lactose-Monohydrat | 10039-26-6 | 0,400 |
| | Menthol | 2216-51-5 | 0,276 |
| | Petrolatum | 8009-03-08 | 0,271 |
| | Natrium-bicarbonat | 144-55-8 | 0,240 |
| | Natriumchlorid | 7647-14-5 | 0,200 |
| | Kaliumhydrogen-Monopersulfat | 70693-62-8 | 0,100 |
| | Citrus Limon | 8008-56-8 | 0.066 |
| | Natriumcarbonat | 144-55-8 | 0,060 |
| | Pfefferminz Aroma | - | 0,040 |
| | Menthyl-Lactat | 59259-38-0 | 0,031 |

Aromastoffe, insbesondere z.B. Pfefferminzaroma, Menthyl-Lactat, Citrus Limon, Menthol, und Gemische davon, sind optional bzw. ersetzbar.

Die Paste zeichnet sich vorzugsweise durch die folgenden Eigenschaften aus:
1. Haftungseigenschaften getestet nach ISO Norm 10873:2010 Zahnheilkunde - Haftmittel Abschnitt 7.3 Stabilitätsbestimmung - Alterungsprozedur (Dentistry - Denture adhesive section 7.3 Determination of stability - Aging procedure) (Bedingungen: Test (peel and adhesion test) (a) 1 min, 25°C, (b) 10 min, 25°C). Haftfestigkeit (adhesion strength): (a) > 7,6 N
   (b) > 7,6 N.
2. Viskositätsmessung mit Brookfield-Viskosimeter (Bedingungen: 5 g Propylenglycol, 1 g der erfindungsgemässen Paste; 100 RPM mit Spindel 4 bei 25°C). Viskosität: 35 ± 5 mPa s.
3. Konsistenzmessung nach BE EN 30193-1:1994 (dentistry standard for resilient lining materials for removable dentures), Bedingungen: Gewicht 1 kg, 25°C. Wert für die Konsistenz: 20-40 mm (d.h. der durchschnittliche Ausbreitungsdurchmesser (in Millimeter) einer Menge von 1,5 g der zu prüfenden Creme zwischen zwei Glasplatten nach 5 Minuten, wobei die obere Platte mit einem Gewicht von 1 kg beladen ist).

### Anwendungszusammensetzung B:

Die folgende Tabelle zeigt ein erfindungsgemässes Mittel in Form eines Pulvers zur topischen Anwendung.

| **Inhaltsstoffe** | | **CAS Nr.** | **Gewichtsprozent (Gew.-%)** |
|---|---|---|---|
| | Zitronensäure | 77-92-9 | 30 |
| | Natriumlaurylsulfat | 151-21-3 | 15 |
| | Natrium-bicarbonat | 144-55-8 | 20 |
| | Natriumchlorid | 7647-14-5 | 10 |
| | Kaliumhydrogen-Monopersulfat | 70693-62-8 | 5 |
| | Bindemittel | | 20 |

### Beispiel 1 (Vergleichsbeispiel):

| | |
|---|---|
| Befund: | Hautriss, ungefähr 2 mm weit, 10 mm lang und 2-3 mm tief, am rechten Ferse. |
| Behandlung: | Je drei Anwendungen der Zusammensetzung gemäss Anwendungszusammensetzung A an fünf konsekutiven Tagen auf die Ferse. |
| Methode: | Die aufgebrachte Creme wurde nach dem Auftragen mit Wasser überstrichen. |
| Heilungsprozess: | Sofortige Schmerzlinderung. Nach fünf Tagen war der Hautriss vollständig abgeheilt. |

### Beispiel 2 (Vergleichsbeispiel):

| | |
|---|---|
| Befund: | Seit 20 Jahren verhärtete, raue, trockene, rissige Haut an den Fersen mit mehreren Fissuren einer Tiefe von ca. 2 mm. |
| Behandlung: | Je drei Anwendungen der Zusammensetzung gemäss Anwendungszusammensetzung A an vier konsekutiven Tagen (insgesamt also 12 Anwendungen) auf die Fersen. |
| Methode: | Die Creme wurde nach dem Auftragen mit einem Verband abgedeckt. |

| | |
|---|---|
| Heilungsprozess: | Am vierten Tag waren die Verhärtungen verschwunden und die Fissuren geschlossen und weitestgehend abgeheilt. |

### Beispiel 3 (Vergleichsbeispiel):

| | |
|---|---|
| Allg. Zustand: | Ein diabetischer Patient, der schon all seine Finger verlor. |
| Befund: | Diabetischer Patient mit einer Operationswunde (mit einem Durchmesser von ca. 2-3 mm) an seinem linken Fuss nach Amputation eines Zehens. Regelmässiges Reinigen der Wunde mit einem Desinfektionsmittel und anschliessendes Verbinden. Während mehr als vier Wochen nach der Operation hat sich noch keine Heilung eingestellt. Die Wunde ist infiziert, Eiter entweicht. |
| Behandlung: | Eine einzige Anwendung der Anwendungszusammensetzung B (in Pulverform). |
| Methode: | Reinigen der Wunde mit einem Desinfektionsmittel und bestreuen der Wunde mit dem Pulver (ca. 2-3 g der Anwendungszusammensetzung B). Für zwei Tage Abdecken der Wunde mit einem Verband. Ab dem zweiten Tag nach der einmaligen Anwendung der Anwendungszusammensetzung B regelmässiges Reinigen mit einem Desinfektionsmittel. |
| Heilungsprozess: | Direkt nach der Anwendung spürte der Patient keine Irritation. Am zweiten Tag nach der Anwendung wurde das Abfliessen einer dunklen Flüssigkeit beobachtet. Gleichzeitig spürte der Patient eine Irritation. An den folgenden Tagen hat sich die Wunde sukzessive geschlossen. |

### Beispiel 4 (Vergleichsbeispiel):

| | |
|---|---|
| Befund: | Schmerzhafte Hautschwiele am linken Daumen |
| Behandlung: | Je eine Anwendung einer Creme gemäss Anwendungszusammensetzung A an zwei konsekutiven Tagen. |

| | |
|---|---|
| Methode: | Die Creme wurde nach dem Auftragen mit einem Haftpflaster abgedeckt. |
| Heilungsprozess: | Der Schmerz war nach der ersten Nacht weg. Nach zwei Tagen war die Hautschwiele verschwunden. |

### Beispiel 5 (Vergleichsbeispiel):

| | |
|---|---|
| Befund: | Schmerzhafte Hautschwiele am rechten Daumen |
| Behandlung: | Je eine Anwendung einer Creme gemäss Anwendungszusammensetzung A an vier konsekutiven Tagen. |
| Methode: | Die Creme wurde nach dem Auftragen mit einem Haftpflaster abgedeckt. |
| Heilungsprozess: | Der Schmerz war nach 3 Tagen weg. Nach einer Woche war die Hautschwiele deutlich kleiner. |

### Beispiel 6 (Vergleichsbeispiel):

| | |
|---|---|
| Befund: | Feiner Hautriss einer Länge von ca. 5-7 mm in der Faltung des Ohrmuschelrands des linken Ohrs |
| Behandlung: | Eine einzige Anwendung der Creme gemäss Anwendungszusammensetzung A. |
| Methode: | Auftragen der Creme gemäss Anwendungszusammensetzung A und Überstreichen der aufgebrachten Creme mit einem nassen Finger. |
| Heilungsprozess: | Sofortige Schmerzlinderung. Nach ca. 12 Stunden ist die Creme oberflächlich ausgetrocknet und deckt die Stelle mit dem Hautriss ab. Nach ca. 24 Stunden wurde die verbliebene Creme abgewaschen. Der Hautriss war nicht mehr zu erkennen also weitestgehend abgeheilt. |

### Beispiel 7:

| | |
|---|---|
| Befund: | Patienten mit oder ohne chronischer Parodontitis. |
| Behandlung: | Eine Anwendung der Anwendungszusammensetzung A nach einer regelmässig durchgeführten Zahnreinigungsbehandlung, insbesondere nach einer Zahn- und Taschenreinigung, insbesondere nach einer Scaling-and-Root-Planing-(SRP)-Behandlung. |
| Methode: | Auftragen der Creme gemäss Anwendungszusammensetzung A auf das Zahnfleisch, insbesondere auf den Zahnfleischrand. |
| Ergebnis: | Die Anwendungszusammensetzung A lindert Schmerzen und fördert den Heilungsprozess nach der Behandlung (insbesondere von während der Behandlung entstandenen kleinen Verletzungen), schützt für einige Zeit nach der Behandlung, indem sie die behandelten Stellen abdeckt und reduziert oder verhindert Zahnfleischbluten nach der Behandlung. |

### Beispiel 8:

Studie zur Evaluierung der biofilmhemmenden und biofilmzerstörenden Aktivität am Beispiel der Anwendungszusammensetzung A.

Ziel dieser Studie war es, die Biofilm inhibierende und Biofilm zerstörende Aktivität einer Salbe gegen *Staphylococcus aureus* zu beurteilen. Um dieses Ziel zu erreichen, wurde ein *In-vitro*-Modellsystem benutzt, das die in Wunden *in vivo* gefundenen Bedingungen nachahmt.

### a) Materialien und Methoden:

### 1. Stämme und Kulturbedingungen

Staphylococcus aureus Mu50 wurde auf Luria Bertani (LB) Agar (BD, Sparks, MD) bei 37°C aerob gezüchtet. S. aureus Mu50 ist ein Methicillin-resistenter S. aureus Stamm.

### 2. Getestetes Produkt

Die Salbe A (d.h. Anwendungszusammensetzung A) wurde durch Verteilen von etwa 250 mg Aliquoten auf der künstlichen Dermis beurteilt.

### 3. Herstellung der künstlichen Dermis

Die künstliche Dermis setzt sich aus einer oberen Schicht und einer unteren Schicht zusammen. Die obere Schicht ist eine chemisch vernetzte, schwammige Hyaluronsäure-(HA)-Lage und wurde wie zuvor beschrieben mit geringfügigen Modifikationen hergestellt (Mineo et al, 2012). Natriumhyaluronatpulver (1,20-1,80 MDA, Lifecore biomedical, MN, US) wurde in destilliertem Wasser (DW) bei einer Konzentration von 1,5 % (pH 6,8) aufgelöst. Diese hochmolekulare HA-(HMWA)-Lösung wurde mit einer verdünnten HCl-Lösung auf einen pH-Wert von 3,5 eingestellt. Ethylenglykoldiglycidylether (EX810; Sigma Aldrich, Bornem, Belgien) wurde als Vernetzungsmittel für HA-Moleküle benutzt. Eine wässrige EX810-Lösung wurde der HMW-HA-Lösung unter kräftigem Rühren tropfenweise zugegeben (Gewichtsverhältnis HA zu EX810: 5-1). Diese Mischlösung wurde in einen Gefriertrocknungscontainer gegossen, bei 4°C über Nacht gelagert, bei -80°C gefroren und dann gefriergetrocknet. Dieser Schwamm wurde sorgfältig mit DW gespült, um eventuelles freies Vernetzungsmittel zu entfernen, dann bei -80°C gefroren und gefriergetrocknet, um die schwammige Vernetzungs-HMW-HA-Lage (cHMW-HA) zu erhalten. Die untere Schicht ist eine schwammige Lage bestehend aus HA und Kollagen (Col). HA-Pulver wurde in DW bei 1 % Konzentration aufgelöst, um HMW-HA-Lösung (pH 6,8) herzustellen. Separat wurde HA (1 %) bei 120°C für 1 h autoklaviert, um eine teilhydrolysierte niedermolekulare HA-(LMW-HA)-Lösung zu erhalten. Es wurde eine 0,1 % Col enthaltende Kollagenlösung hergestellt, bei 50°C für 10 min erwärmt, um eine hitzedenaturierte Kollagenlösung zu erhalten. Die oben genannten drei Lösungen wurden gemischt und dann mit NaOH auf pH 7,5 eingestellt. Diese Mischlösung wurde in einen Gefriertrocknungscontainer gegossen, in dem die schwammige cHMW-HA-Lage eingetaucht war. Das kombinierte Produkt wurde bei 4°C über Nacht gelagert, dann bei -80°C gefroren und gefriergetrocknet, um eine zweischichtige schwammige Lage zu erhalten. Beide Seiten der schwammigen Lage wurden mit einer UV-Lampe 20 min lang bestrahlt, um die Kollagenmoleküle zu vernetzen. Diese schwammige Lage wurde bei 100°C für 1 h sterilisiert, um eine sterile künstliche Dermis (AD) zu erhalten.

### 4. Chronisches In-vitro-Wundmodell zum Beurteilen der inhibitorischen und Biofilm zerstörenden Wirkung der Salbe

Biofilme wurden in einem chronischen pathogenen Wund-Biofilmmodell gezüchtet. Eine Übernacht-Kultur des getesteten Stamms wurde pelletiert, gewaschen, in physiologischer Kochsalzlösung (PS) resuspendiert und auf 106 CFU ml⁻¹ (Koloniebildungseinheiten pro Milliliter) verdünnt. Die AD wurde in 0,5 ml chronischem Wund-Biofilmmedium (Bolton Broth mit 50 % Plasma und 5 % haemolysiertem Frost-Tau-Pferdeblut und 10U/ml Heparin) eingeweicht und in die Kavitäten (englisch *wells*) einer 24-Well-Mikrotiterplatte (TPP) gegeben, und 10 µl Aliquoten der Zellsuspension wurden auf jede AD getupft. Jeder Kavität (d.h. jedem *well*) wurden 0,5 ml chronisches Wund-Biofilmmedium zugegeben. Um die Biofilm inhibierende Wirkung der Salbe zu beurteilen, wurden 250 mg Aliquote unmittelbar nach der Inokulation auf die AD gegeben und das Biofilmmodell wurde 24 h lang bei 37°C inkubiert, um eine Biofilmbildung auf der AD zuzulassen.

Um die Biofilm zerstörende Aktivität der Salbe zu beurteilen, wurde das Biofilmmodell 24 h lang bei 37°C inkubiert, um eine Biofilmbildung auf der AD zuzulassen. Nach dieser Biofilmbildung wurde das Medium entfernt und die Biofilme mit PS gewaschen, frisches Medium wurde zugegeben und Salbe wurde auf den Biofilm gegeben.

### 5. Plattierung und Datenanalyse

In beiden Experimententypen wurden Biofilme nach der Behandlung mit PS gespült, um die Behandlung zu entfernen. Die AD wurde in 10 ml PS gegeben. Sessile Zellen wurden mit drei Zyklen von Verwirbeln (30s) und Beschallen (30 s; Branson 3510; Branson Ultrasonics Corp., Danbury, CT) von der AD entfernt, und die Zahl an CFU/AD (/ml) wurde durch Plattieren der resultierenden Suspensionen ermittelt. Die normale Verteilung der Daten wurde mit dem Shapiro-Wilk-Test geprüft. Die Daten wurden durch eine unabhängige statistische t-Test-Probenanalyse analysiert. Statistische Analyse und lineare Regressionsanalyse wurden mit SPSS-Software, Version 22.0 (SPSS, Chicago, IL, USA) durchgeführt.

### b) Ergebnisse:

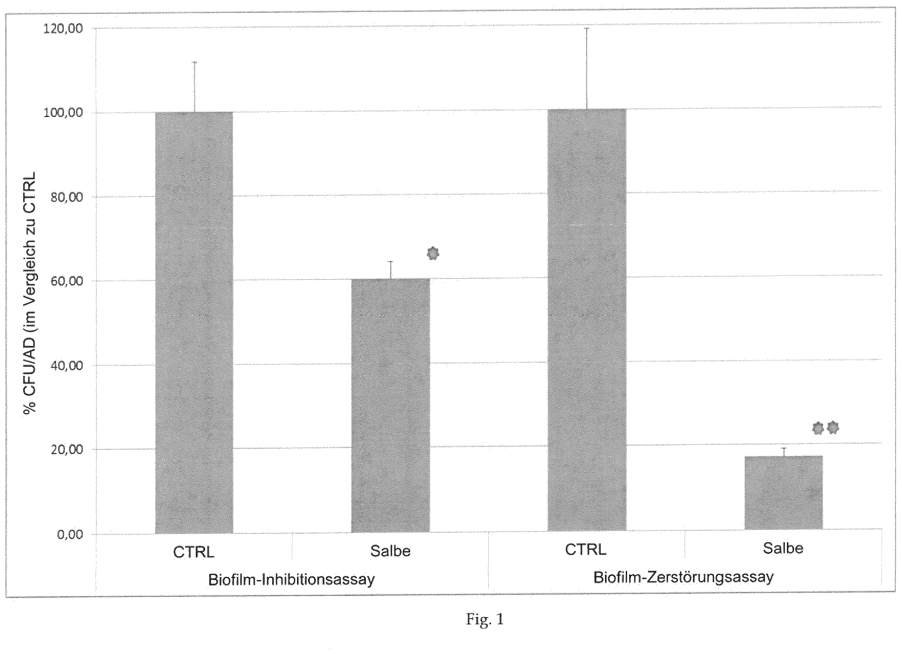

Die Biofilm inhibierende und Biofilm zerstörende Wirkung der Salbe gegen S. aureus Mu50 ist in Fig. 1, Tabelle 1 und Tabelle 2 dargestellt. Die Salbe zeigte eine niedrige, aber doch signifikante (p<0,01) Biofilm inhibierende Wirkung. Die Behandlung mit der Salbe während der Biofilmbildung führte zu einer 40%igen Reduzierung der Anzahl an CFU/AD. Zusätzlich zeigte die Salbe eine mäßige Biofilm zerstörende Aktivität. Die Behandlung eines maturen Biofilms mit der Salbe führte zu einer 82,8%igen Reduzierung der Anzahl an CFU/AD.

**Tabelle 1. Inhibitorische Wirkung der Salbe auf S. aureus Mu50**

| **Behandlung** | **CFU/AD** | | **Log CFU/AD** | | **% CFU/AD** | |
|---|---|---|---|---|---|---|
| | **Ø** | **Std.Abw.** | **Ø** | **Std.Abw.** | **Ø** | **Std.Abw.** |
| CTRL (ohne Behandlung) | 1,87E+08 | 2,20E+07 | 8,27 | 0,05 | 100,0 | 11,8 |
| Salbe | 1,12E+08* | 8,00E+06 | 8,05* | 0,03 | 60,0* | 4,29 |

| | | | | | | |
|---|---|---|---|---|---|---|
| *unterscheidet sich signifikant von unbehandelter Kontrolle (p < 0,01) | | | | | | |

Die Daten in der Tabelle sind als Mittel (CFU/AD) ± Standardabweichung, mittlere Log-Werte (CFU/ AD) ± Standardabweichung und mittlerer %-Wert CFU/ AD (im Vergleich zu CTRL) angegeben.

**Tabelle 2. Biofilm zerstörende Wirkung der Salbe auf S. aureus Mu50.**

| **Behandlung** | **CFU/AD** | | **Log CFU/AD** | | **% CFU/AD** | |
|---|---|---|---|---|---|---|
| | **Ø** | **Std.Abw.** | **Ø** | **Std.Abw.** | **Ø** | **Std.Abw.** |
| CTRL (ohne Behandlung) | 2,13E+08 | 4,11E+07 | 8,33 | 0,08 | 100,0 | 19,2 |
| Salbe | 3,67E+07* | 4,04E+06 | 7,56* | 0,05 | 17,2* | 1,9 |

| | | | | | | |
|---|---|---|---|---|---|---|
| * unterscheidet sich signifikant von unbehandelter Kontrolle (p < 0,01) | | | | | | |

Die Daten in der Tabelle sind als Mittel (CFU/ AD) ± Standardabweichung, mittlere Log-Werte (CFU/ AD) ± Standardabweichung und mittlerer %-Wert CFU/ AD (im Vergleich zu CTRL) angegeben.

Die Ergebnisse werden ferner in Fig. 1 dargestellt. Fig. 1 präsentiert die Biofilm inhibierende und zerstörende Wirkung der Salbe. Daten sind als mittlerer %-Wert CFU/AD (im Vergleich zur unbehandelten CTRL) ± Standardabweichung angegeben. In dem Biofilm-Inhibitionsassay führte das Produkt zu einem signifikanten (p<0,05) Rückgang der Biofilmbildung im Vergleich zur unbehandelten Kontrolle (beschriftet mit CTRL) (* in Fig. 1). Im Biofilm-Zerstörungsassay führte das Produkt zu einem signifikanten (p>0,005) Rückgang eines maturen Biofilms im Vergleich zur unbehandelten Kontrolle (beschriftet mit CTRL) (** in Fig. 1).

### Beispiel 9:

### Multizentrische, offene, randomisierte klinische Studie

Durchgeführt wurde eine multizentrische, offene, randomisierte klinische Studie zum Beurteilen und Vergleichen von Wirksamkeit und Sicherheit des erfindungsgemäßen Mittels in Form eines Gingival-Pastendressing gemäß Anwendungszusammensetzung A während einer Testphase I und in Form einer Zahnfleischbürstlösung, hergestellt aus der Anwendungszusammensetzung B, während einer Testphase II, wobei beide Formen des erfindungsgemäßen Mittels bei Probanden, die an chronischer Parodontitis litten, als Zusatzanwendung nach einer vorausgegangenen Scaling und Wurzelglättung (SRP) zur Anwendung kamen.

### 9.1. Probandenbeschreibungen:

Es wurden insgesamt 29 männliche und weibliche Probanden bzw. Patienten (Alter 30-50), die an chronischer Parodontitis litten und einer SRP-Behandlung bedurften, untersucht und für die Studie registriert. Von diesen Probanden wurden 20 Probanden in einer Behandlungsgruppe und 19 Probanden einer Kontrollgruppe randomisiert.

### 9.2. Behandlung:

### Behandlung der Behandlungsgruppe:

Behandelt mit Scaling und Wurzelglättung (SRP), gefolgt von Phase I und Phase II:
Phase I: Unmittelbar nach der SRP-Behandlung an Probanden, die an chronischer Parodontitis litten, wurde die Zahnfleischpaste (Anwendungszusammensetzung A) einmal über das Zahnfleisch und den Gingival-Sulcus für 2 bis 3 Stunden aufgebracht.
Phase II: Auf Phase I folgte das Bürsten von Zähnen und Zahnfleisch mit der Zahnfleischbürstlösung einmal täglich jeden Abend nach der letzten Mahlzeit des Tages an 10 aufeinander folgenden Tagen, beginnend am Tag nach der SRP-Behandlung. Die Zahnfleishbürstlösung wurde jeden Tag frisch aus der Anwendungszusammensetzung B hergestellt: eine Tablette (etwa 2 g) der Anwendungszusammensetzung B wurde in etwa 15 ml Wasser unmittelbar vor dem Gebrauch aufgelöst.

Die Dauer der Behandlung, einschließlich Phase I und Phase II, betrug 11 Tage. Behandlung der Kontrollgruppe:
Nur mit Scaling und Wurzelglättung (SRP) behandelt.

### 9.3. Untersuchungsplan:

Beide Behandlungsgruppen wurden an Tag 0, Tag 5 und Tag 11 wie folgt beurteilt:
- vor SRP-Behandlung an Tag 0 (oder bis zu 5 Tagen früher): demografische Daten, körperliche Untersuchung, Vitalparameter, Leistungsparameter,
- an Tag 0 nach SRP-Behandlung (Kontrollgruppe) oder nach Phase-I-Behandlung (Behandlungsgruppe): körperliche Untersuchung, Vitalparameter, Leistungsparameter,
- an Tag 5: körperliche Untersuchung, Vitalparameter, klinische parodontale Parameter und Leistungsparameter,
- an Tag 11: körperliche Untersuchung, Vitalparameter, klinische parodontale Parameter und Leistungsparameter, Gesamtbakterienzahl und Analyse, Wirksamkeit wurde durch Ermitteln von subgingivaler Plaque beurteilt.

Bei jeder Beurteilung gemessene Parameter (Leistungsparameter):
- Plaque-Index (PI)
- Gingival-Index (Gl)
- parodontaler Blutungsindex (PBI)

Bakterienzahl von parodontophtogenen Bakterien:
- Prophyrompnas gingivalis (Pg)
- Prevotella intermedoa (Pi)
- Aggregatibacer actinomycetemcomitans (Aa)
- Fusobacterium nucleatum (Fn)

### 9.4. Testergebnisse

### 9.4.1. Mikrobielle Zahnfleischinfektion über die Behandlungsperiode:

Kontrollgruppe: 66,7 % waren zu Beginn der Behandlung infiziert. Von diesen 66,7 % waren 100 % auch nach 11 Tagen noch infiziert.

Behandlungsgruppe: 60 % waren zu Beginn der Behandlung infiziert. Von diesen 60 % waren nur noch 25 % nach 11 Tagen infiziert.

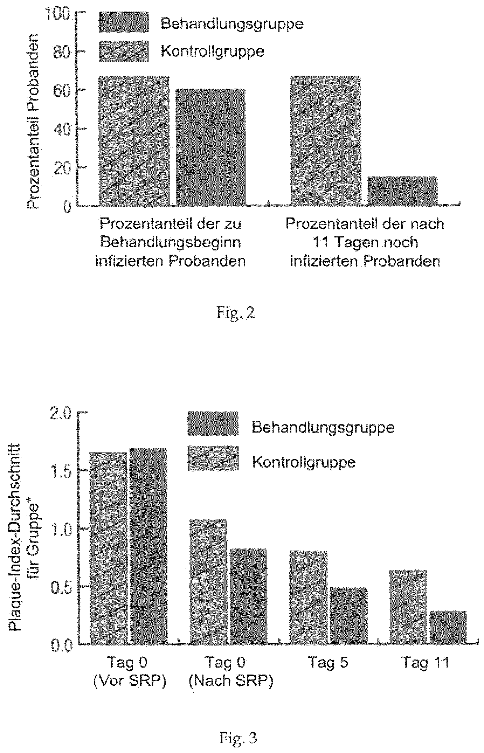

Bei einem Vergleich der Kontrollgruppe und der Behandlungsgruppe zeigt Fig. 2 auf der linken Seite des Diagramms den Prozentanteil von Probanden jeder Gruppe, die zu Beginn der Behandlung infiziert waren, und auf der rechten Seite des Diagramms den Prozentanteil von Probanden jeder Gruppe, die auch an Tag 11 noch infiziert waren.

Nach voller Behandlung an Tag 11 war eine große Zahl der Probanden der Behandlungsgruppe infektionsfrei, während die Kontrollgruppe weiterhin dieselbe Anzahl an infizierten Probanden zeigte wie zu Beginn der Behandlung.

### 9.4.2. Zusammenfassung der Plaque-Index-Progression über die Behandlungsperiode:

Bei einem Vergleich der Kontrollgruppe und der Behandlungsgruppe zeigt Fig. 3 den Plaque-Index-Durchschnitt für jede Gruppe an Tag 0 vor SRP, an Tag 0 nach SRP und Phase-I-Behandlung, an Tag 5 und an Tag 11.

Die Plaque-Niveaus wurden mit der Turkesy-Modifikation des Quiglet-Hein Plaque Index (* in Fig. 3) beurteilt.

Während der Behandlungsperiode von 11 Tagen nahm das Plaque-Niveau in beiden Gruppen ab, aber in der Behandlungsgruppe auf ein deutlich niedrigeres Niveau als in der Kontrollgruppe.

### 9.4.3. Zusammenfassung der Gingival-Index-Progression über die Behandlungsperiode

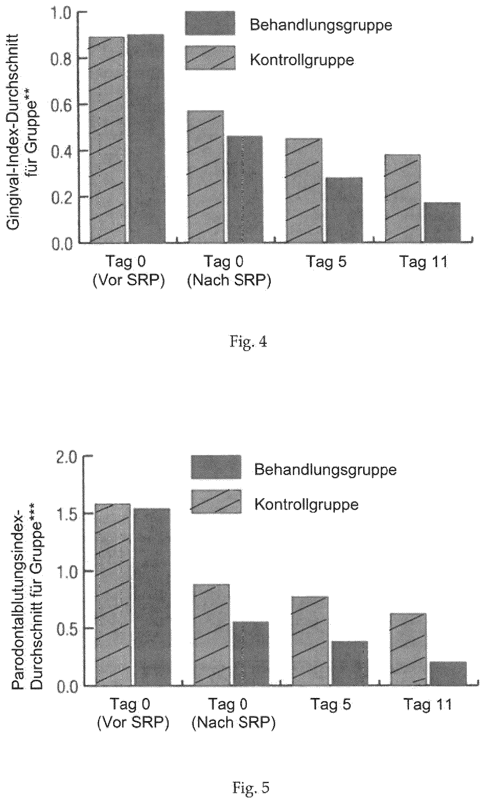

Bei einem Vergleich der Kontrollgruppe und der Behandlungsgruppe zeigt Fig. 4 den Gingival-Index-Durchschnitt für jede Gruppe an Tag 0 vor SRP, an Tag 0 nach SRP und Phase-I-Behandlung, an Tag 5 und an Tag 11.

Der Gingival-Index wurde mit dem Loe and Silness 1963 Indexsystem zur Beurteilung des Gingival-Zustands beurteilt, qualitative Veränderungen der Gingiva wurden aufgezeichnet (** in Fig. 4). Es wurden die marginalen und interproximalen Gewebe separat auf der Basis von 0 bis 3 bewertet.

Während der Behandlungsperiode von 11 Tagen nahm das Gingival-Niveau in beiden Gruppen ab, aber in der Behandlungsgruppe auf ein deutlich niedriges Niveau als in der Kontrollgruppe.

### 9.4.4. Zusammenfassung der parodontalen Blutungsindexprogression über die Behandlungsperiode

Bei einem Vergleich der Kontrollgruppe und der Behandlungsgruppe zeigt Fig. 5 den parodontalen Blutungsindexdurchschnitt für jede Gruppe an Tag 0 vor SRP, an Tag 0 nach SRP und Phase-I-Behandlung, an Tag 5 und an Tag 11.

Der parodontale Blutungsindex wurde mit dem Muhleman and Son 1971 Score-Chart gemessen, der die Sulcus-Blutung beschreibt (*** in Fig 5).

Während der Behandlungsperiode von 11 Tagen nahm das parodontale Blutungsniveau in beiden Gruppen ab, aber in der Behandlungsgruppe auf ein deutlich niedrigeres Niveau als in der Kontrollgruppe.

### 9.4.5. Halitosis-Gefühl nach 11 Tagen

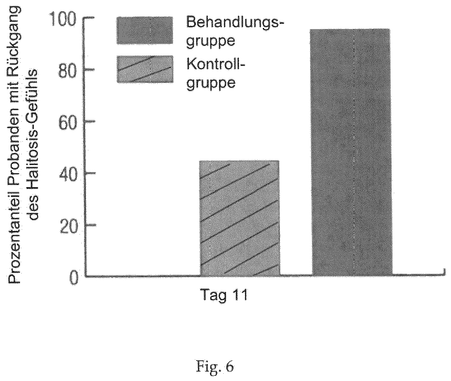

Bei einem Vergleich der Kontrollgruppe und der Behandlungsgruppe zeigt Fig. 6 den Prozentanteil an Probanden, die einen Rückgang des Halitosis-Gefühls erfuhren, für jede Gruppe an Tag 11.

100 % der Probanden der Behandlungsgruppe erfuhren einen Rückgang des Halitosis-Gefühls, während in der Kontrollgruppe nur etwa 40 % der Probanden einen Rückgang erfuhren.

### 9.5 Sicherheitsergebnisse:

Es wurden während der Studienperiode keine ernsthaften nachteiligen Ereignisse gemeldet. Keiner der Probanden wurde aufgrund eines nachteiligen Ereignisses aus der Studie ausgeschlossen. Die Gesamttestbehandlung wurde von allen Studienprobanden gut toleriert.

### 9.6. Schlussfolgerung:

Keiner der Probanden der Behandlungsgruppe beschwerte sich nach dem Aufbringen des parodontalen Pastendressing über eine Reizung. Gruppeninterne Vergleiche zeigten, dass die Probanden der Behandlungsgruppe statistisch signifikant größere Rückgänge von Plaque-Index, Gingival-Index und parodontalem Blutungsindex nach 11 Tagen erfuhren.

Das Aufbringen des parodontalen Pastendressing reduzierte die Bakterienbelastung des Zahnfleischs im Vergleich zur Kontrollgruppe an Tag 11 erheblich. Insgesamt 9 von 11 Probanden (82 %) in der Behandlungsgruppe waren nach 11 Behandlungstagen erfolgreich von einer Bakterieninfektion geheilt, während in der Kontrollgruppe keiner der Probanden nach Tag 11 geheilt war.

Nach der Behandlung erfuhren alle Probanden der Behandlungsgruppe einen Rückgang von Zahnfleischschmerzen, Trockenheitsgefühl, Zahnfleisch- oder Sulcus-Blutung, einschließlich Infektionssymptomen. Zusammenfassend, die Benutzung von parodontalem Pastendressing und Zahnfleischbürstlösung als Ergänzung zur SRP-Behandlung ist für Probanden gegenüber solchen, die nur SRP-Behandlung erhalten, äußerst nützlich.

Insgesamt zeigen diese Ergebnisse von Beispiel 9, dass das Gingival-Pastendressing und die Zahnfleischbürstlösung als Ergänzung zu SRP beim Management von chronischer Parodontitis wirksam und sicher sind und gut toleriert werden.

Während vorstehend spezifische Ausführungsformen beschrieben wurden, ist es offensichtlich, dass unterschiedliche Kombinationen der aufgezeigten Ausführungsmöglichkeiten angewendet werden können, insoweit sich die Ausführungsmöglichkeiten nicht gegenseitig ausschliessen.

Während die Erfindung vorstehend unter Bezugnahme auf spezifische Ausführungsformen beschrieben wurde, ist es offensichtlich, dass Änderungen, Modifikationen, Variationen und Kombinationen ohne vom Erfindungsgedanken abzuweichen gemacht werden können.

## Patentansprüche

1. Mittel zur Verwendung bei der Behandlung von Parodontitis, beinhaltend wenigstens eine salzförmigen Chloridverbindung ausgewählt aus der Gruppe der Alkali- oder Erdalkalimetallsalze und wenigstens ein Oxidationsmittel ausgewählt aus der Gruppe der Persulfatverbindungen.

2. Mittel nach Anspruch 1, zur Verwendung
- bei der Behandlung der marginalen Parodontitis oder als Zusatzbehandlung bei der apikalen Parodontitis, und/oder
- bei der Behandlung von Parodontitis im Anschluss an eine Zahnreinigungsbehandlung, insbesondere im Anschluss an eine Scaling-and-Root-Planing-(SRP)-Behandlung.

3. Mittel zur Verwendung nach einem der Ansprüche 1 oder 2, **dadurch gekennzeichnet, dass** das Mittel in Form eines pulverförmigen Feststoffgemisches vorliegt und als Pulver zur Anwendung bereitgestellt wird oder
- das Mittel in Form eines pulverförmigen Feststoffgemisches einer Haftungszusammensetzung beigemischt ist und als Paste, Creme oder Gel zur Anwendung bereitgestellt wird.

4. Mittel zur Verwendung nach einem der Ansprüche 1 bis 3, **dadurch gekennzeichnet, dass** als Oxidationsmittel aus der Gruppe der Persulfatverbindungen wenigstens eine Hydrogenperoxosulfatverbindung, wie z.B. Kaliumhydrogenmonopersulfat (KHSOs), enthalten ist.

5. Mittel zur Verwendung nach einem der Ansprüche 1 bis 4, **dadurch gekennzeichnet, dass** die wenigstens eine salzförmige Chloridverbindung ausgewählt ist aus der Gruppe der Alkalisalze.

6. Mittel zur Verwendung nach einem der Ansprüche 1 bis 5, **dadurch gekennzeichnet, dass** als die wenigstens eine salzförmige Chloridverbindung Kochsalz (NaCl) enthalten ist.

7. Mittel zur Verwendung nach einem der Ansprüche 1 bis 6, **dadurch gekennzeichnet, dass** das Mittel eine Carbonsäure, vorzugsweise eine Mono- oder Dicarbonsäure wie Zitronensäure, enthält.

8. Mittel zur Verwendung nach Anspruch 7, **dadurch gekennzeichnet, dass** die Säure in einer solchen Menge enthalten ist, dass das in einer bestimmten Menge einer wässerigen Lösung aufgelöste Mittel einen pH-Wert < 6, vorzugsweise < 5, und ganz besonders bevorzugt < 4.5 erzeugt.

9. Mittel zur Verwendung nach einem der Ansprüche 1 bis 8, **dadurch gekennzeichnet, dass** das Oxidationspotential des Oxidationsmittels in wässeriger Lösung mindestens im beanspruchten pH-Bereich höher als das Oxidationspotential von Cl¹⁻/Cl^{o} ist.

10. Mittel zur Verwendung nach einem der Ansprüche 1 bis 9, **dadurch gekennzeichnet, dass** Stoffe zur Lösungsbeschleunigung (Brausesalze), beispielsweise eine Carbonat oder Bicarbonat enthaltende Verbindung wie z.B. Natriumcarbonat oder Natriumbicarbonat, und eine mindestens stöchiometrische Menge Säure enthalten sind.

11. Mittel zur Verwendung nach einem der Ansprüche 1 bis 10, **dadurch gekennzeichnet, dass** ein Bindemittel, und optional Aroma-, Farb- und Hilfsstoffe, wie z.B. Stoffe zur Wasserenthärtung, Füllstoffe und dergleichen, enthalten sind.

12. Mittel zur Verwendung nach einem der Ansprüche 1 bis 11, **dadurch gekennzeichnet, dass** keine Fluoridsalze, vorzugsweise keine Fluoridverbindungen, enthalten sind.

13. Mittel zur Verwendung nach einem der Ansprüche 1 bis 12, **dadurch gekennzeichnet, dass** zur Verwendung bei Behandlung das Mittel topisch aufgebracht wird.

## Claims

1. An agent for use in the treatment of periodontitis containing at least one salt-type chloride compound selected from the group consisting of alkali or alkali earth metal salts and at least one oxidizing agent selected from the group of persulfate compounds.

2. An agent according to claim 1, for use
- in the treatment of marginal periodontitis or as additional treatment of apical periodontitis and/or
- in the treatment of periodontitis subsequent to dental cleaning treatment, in particular subsequent to a scaling and root planning (SRP) treatment.

3. An agent for use according to one of the claims 1 or 2, **characterized in that** the agent is available as a powdery solid mixture and is made available as a powder for use or
the agent is mixed as a powdery solid mixture to an adhesive compound and is made available as a paste, a cream or a gel for use.

4. An agent for use according to one of the claims 1 to 3, **characterized in that** at least one hydrogen peroxosulfate compound such as, for example, potassium hydrogen monopersulfate (KHSOs) is contained as an oxidizing means from the group of the persulfate compounds.

5. An agent for use according to one of the claims 1 to 4, **characterized in that** the at least one salt-type chloride compound is selected from the group of the alkali salts.

6. An agent for use according to one of the claims 1 to 5, **characterized in that** table salt (NaCl) is contained as at the least one-type chloride compound.

7. An agent for use according to one of the claims 1 to 6, **characterized in that** the agent contains a carboxylic acid, preferably a mono- or dicarboxylic acid such as citric acid.

8. An agent for use according to claim 7, **characterized in that** the acid is contained in such a quantity that the agent dissolved in a certain quantity of an aqueous solution produces a pH value < 6, preferably < 5 and very particularly preferably < 4.5.

9. An agent for use according to one of the claims 1 to 8, **characterized in that** the oxidation potential of the oxidizing agent in an aqueous solution is at least in the claimed pH range higher than the oxidation potential of Cl⁻¹/Cl^{o}.

10. An agent for use according to one of the claims 1 to 9, **characterized in that** substances for acceleration of dissolution (effervescent salts), for example a carbonate or bicarbonate containing compound such as sodium carbonate or sodium bicarbonate, and an at least stoichiometric quantity of acid are contained.

11. An agent for use according to one of the claims 1 to 10, **characterized in that** a binding agent and optionally aromatic, coloring and excipients such as, for example, substances for water softening, fillers and the like are contained.

12. An agent for use according to one of the claims 1 to 11, **characterized in that** no fluoride salts, preferably no fluoride compounds, are contained.

13. An agent for use according to one of the claims 1 to 12, **characterized in that** for use in the treatment the agent is applied topically.

## Revendications

1. Moyen pour l'utilisation lors du traitement de la parodontite contenant au moins un composé de chlorure salé sélectionné dans le groupe des sels de métaux alcalins et alcalino-terreux et au moins un oxydant sélectionné dans le groupe des composés de persulfate.

2. Moyen selon la revendication 1 pour l'utilisation
- lors du traitement de la parodontite marginale ou comme traitement adjuvant lors de la parodontite apicale et/ou
- lors du traitement de la parodontite suite à un traitement de nettoyage dentaire, en particulier suite à un détartrage et surfaçage radiculaire (DSR).

3. Moyen pour l'utilisation selon l'une des revendications 1 ou 2, **caractérisé en ce que** le moyen est sous forme d'un mélange de solides en poudre et est mis à disposition en tant que poudre pour l'application ou
- le moyen sous forme d'un mélange de solides en poudre est mélangé à une composition d'adhérence et est mis à disposition en tant que pâte, crème ou gel pour l'application.

4. Moyen pour l'utilisation selon l'une des revendications 1 à 3, **caractérisé en ce qu'**au moins un composé de peroxodisulfate d'hydrogène comme par exemple du monopersulfate d'hydrogène de potassium (KHSOs) est contenu comme oxydant du groupe des composés de persulfate.

5. Moyen pour l'utilisation selon l'une des revendications 1 à 4, **caractérisé en ce que** le au moins un composé de chlorure salé est sélectionné dans le groupe des sels alcalins.

6. Moyen pour l'utilisation selon l'une des revendications 1 à 5, **caractérisé en ce que** du sel de table (NaCl) est utilisé comme le au moins un composé de chlorure salé.

7. Moyen pour l'utilisation selon l'une des revendications 1 à 6, **caractérisé en ce que** le moyen contient un acide carboxylique, de préférence un acide monocarboxylique ou dicarboxylique comme l'acide citrique.

8. Moyen pour l'utilisation selon la revendication 7, **caractérisé en ce que** l'acide est contenu dans une quantité telle que le moyen dissous dans une certaine quantité de solution aqueuse produit une valeur de pH < 6, de préférence < 5 et de manière tout particulièrement préférée < 4,5.

9. Moyen pour l'utilisation selon l'une des revendications 1 à 8, **caractérisé en ce que** le potentiel d'oxydation de l'oxydant dans une solution aqueuse est plus élevé que le potentiel d'oxydation de Cl¹⁻/Cl^{o} au moins dans la plage de pH revendiquée.

10. Moyen pour l'utilisation selon l'une des revendications 1 à 9, **caractérisé en ce que** des substances pour l'accélération de la dissolution (sels effervescents), par exemple un composé contenant du carbonate ou du bicarbonate comme, par exemple, du carbonate de sodium ou du bicarbonate de sodium et une quantité au moins stoechiométrique d'acide sont contenues.

11. Moyen pour l'utilisation selon l'une des revendications 1 à 10, **caractérisé en ce qu'**un liant et en option des substances aromatiques, colorantes et auxiliaires comme, par exemple, des substances pour adoucir l'eau, des matières de remplissage et équivalent sont contenues.

12. Moyen pour l'utilisation selon l'une des revendications 1 à 11, **caractérisé en ce qu'**aucun sel de fluorure, de préférence aucun composé de fluorure, n'est contenu.

13. Moyen pour l'utilisation selon l'une des revendications 1 à 12, **caractérisé en ce que** le moyen est appliqué de manière topique pour l'utilisation lors du traitement.
